(19) Europäisches Patentamt
European Patent Office
Office européen des brevets

(11) Publication number: 0 445 735 A1

# (12) EUROPEAN PATENT APPLICATION

(21) Application number: 91103315.7

(22) Date of filing: 05.03.91

(51) Int. Cl.5: A61K 7/48, A61K 31/35

(30) Priority: 09.03.90 JP 58449/90

(43) Date of publication of application:
11.09.91 Bulletin 91/37

(84) Designated Contracting States:
DE FR GB IT

(71) Applicant: Sansho Seiyaku Co., Ltd.
26-7, Oike 2-chome
Onojo-shi Fukuoka-ken(JP)

(72) Inventor: Yamamoto, Shinji, c/o Sansho Seiyaku Co.,Ltd.
26-7, Oike 2-chome
Onojo-shi, Fukuoka-ken(JP)

(74) Representative: Patentanwälte Deufel, Hertel, Lewald
Isartorplatz 6 Postfach 26 02 47
W-8000 München 26(DE)

## (54) Melanogenesis-inhibiting endermic preparation.

(57) Disclosed is a melanogenesis-inhibiting endermic preparation for external application which contains, as a skin-whitening active ingredient, a 6-hydroxychroman derivative of a formula:

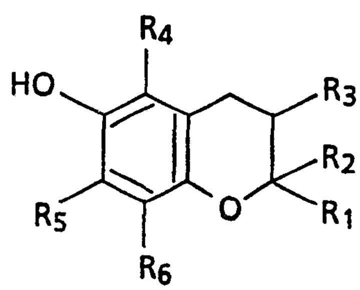

where $R_1$ is H, 1-6C lower alkyl, 1-6C lower alkoxy, $-CO_2R'$, $-(CH_2)_nOH$, $-CH_2CO_2R'$ or -CONHR'; R' is 1-6C lower alkyl; n is 1-6; and $R_2$ to $R_6$ each are H or 1-6C lower alkyl. The preparation has an improved skin-whitening effect and has not harmful side effects.

EP 0 445 735 A1

## BACKGROUND OF THE INVENTION

### Field of the Invention:

The present invention relates to a melanogenesis-inhibiting endermic preparation for external application and, more precisely, to such containing a 6-hydroxychroman derivative as an active ingredient.

### Prior Art:

The feminine desire for the white skin has not changed in ages, and it is well known that various skin-whitening cosmetics and endermic preparations have heretofore been proposed.

From the pathologic and histological viewpoint, melanoderma is said to be caused by an increase of epidermic melanin for example, as seen in pigmentation diseases such as chloasma. Accordingly, skin-whitening endermic preparations generally contain a melanogenesis-inhibiting substance in their compositions.

On the other hand, as for the means of inhibiting melanogenesis in the skin, various methods have been known. For instance, there is the method of continuously taking vitamin C by oral administration, the method of incorporating a light-scattering substance such as talc, zinc flower or titanium oxide, or an ultraviolet inhibitor such as paraaminobenzoic acid into an endermic preparation, the method of incorporating a placenta extract or liver extract into an endermic preparation, and the method of incorporating a kojic acid or kojic acid derivative into an endermic preparation.

As mentioned above, various skin-whitening cosmetic materials containing a melanogenesis-inhibiting substance as an active ingredient as well as various endermic preparations for external application containing the same have heretofore been known. Under this situation, substances having a further effective melanogenesis-inhibiting effect and a high safety factor for human use are desired.

## SUMMARY OF THE INVENTION

The object of the present invention is to provide an endermic preparation for external application which has an effective and sufficient melanogenesis-inhibiting effect.

The present invention has achieved the above-mentioned object, and it is characterized by employment of particular 6-hydroxychroman derivatives as active ingredients.

Specifically, there is provided in accordance with the present invention a melanogenesis-inhibiting endermic preparation containing, as an active ingredient, a 6-hydroxychroman derivative of the general formula:

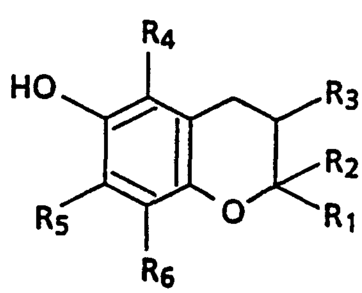

wherein $R_1$ represents a hydrogen atom, a lower alkyl group having from 1 to 6 carbon atoms, a lower alkoxy group having from 1 to 6 carbon atoms, a carboxylate residue of $-CO_2R'$ (wherein R' represents a lower alkyl group having from 1 to 6 carbon atoms), an alcohol residue of $-(CH_2)_nOH$ (wherein n represents an integer of from 1 to 6), an acetate residue of $-CH_2CO_2R'$ (wherein R' represents a lower alkyl group having from 1 to 6 carbon atoms), or a carboxylic acid amide residue of - CONHR' (in which R' represents a lower alkyl group having from 1 to 6 carbon atoms); and $R_2$ to $R_6$ independently represent a hydrogen atom or a lower alkyl group having from 1 to 6 carbon atoms.

## DETAILED DESCRIPTION OF THE INVENTION

Preferred examples of 6-hydroxychroman derivatives of the above-mentioned formula include 2,2,5,7,8-pentamethyl-6-hydroxychroman, 2,2,7,8-tetramethyl-6-hydroxychroman, 2,5,7,8-tetramethyl-2-methoxy-6-hydroxychroman, 2,2,8-trimethyl-5,7-diisopropyl-6-hydroxychroman, 2,5,7,8-tetramethyl-6-hydroxy-2-hydroxymethylchroman, and 2,5,7,8-tetramethyl-6-hydroxychroman.

The endermic preparation of the present invention is used essentially as a skin cosmetic material such as a lotion, cream, emulsion or a pack, or as a skin treatment material such as ointment or cataplasm. The above-mentioned 6-hydroxychroman derivative is incorporated into the base substance of the preparation along with various additives thereto. The content of the 6-hydroxychroman derivative in the preparation is, for example, from 0.001 to 20% by weight, preferably from 0.01 to 10% by weight, as part of the total weight of the preparation.

As preferred embodiments of the melanogenesis-inhibiting endermic preparation of the present invention, there are mentioned a lotion, cream, emulsion, a pack and ointment. Compositions of these preparations will be mentioned in detail hereunder.

Cream:

For the aqueous phase part, a moisturizing agent such as glycerin or sorbitol is incorporated into pure water.

Additionally, a solid oil component such as bee's wax, paraffin, microcrystalline wax, ceresine, higher fatty acid or solidified oil is mixed with a liquid oil component such as squalane, liquid paraffin or various ester oils, and another oily component such as antiseptic or surfactant to prepare an oil phase part, to which is added a determined amount of a 6-hydroxychroman derivative of the above-mentioned formula.

Next, the aqueous phase part is gradually heated to about 80° C, at which point the oil phase part which has been heated up to almost the same temperature is gradually added to the heated aqueous phase part. Then, the resulting mixture is emulsified to form cream.

Emulsion:

For the aqueous phase part, a moisturizing agent such as glycerin and a pH-adjusting agent such as an acid or alkali are added to pure water and mixed with heating.

Additionally, for an oil phase part, an oily component such as antiseptic or surfactant is added to a solid oil component such as bee's wax or paraffin, a semi-solid oil component such as vaseline and lanolin, and a liquid oil component such as squalane, liquid paraffin or various ester oils, and the whole is mixed. Then, a determined amount of a 6-hydroxychroman derivative of the above-mentioned formula is added to the resulting mixture and heated to about 80° C.

Next, the heated oil phase part is added to the previously prepared aqueous phase part, which has been heated to almost the same temperature, and the mixture pre-emulsified. To the resulting pre-emulsion is added a protective colloid such as carboxyvinyl polymer or carboxymethyl cellulose, and the whole is then uniformly emulsified a homogenized to prepare final emulsion.

Pack:

A moisturizing agent such as glycerin and a filming agent such as polyvinyl alcohol or bee gum are added to pure water and are swollen. Then, a powder of kaolin, talc or zinc oxide can be optionally added thereto. Ethyl alcohol containing a perfume or antiseptic is added to the resulting mixture, which is then kneaded to a paste. At any stage of the process, a determined amount of a 6-hydroxychroman derivative of the above-mentioned formula is added to the system.

Ointment:

A hydrophilic component of a moisturizing agent such as glycerin or sorbitol is added to a pure water to prepare an aqueous phase part.

Additionally, an oily phase part is prepared by mixing a 6-hydroxychroman derivative of the above-mentioned formula, a solid oil component such as bee's wax, paraffin, microcrystalline wax, ceresine, higher fatty acid or hardened oil, a semi-solid oil component such as vaseline, lanolin or glyceride, a liquid oil component such as squalane, liquid paraffin or various ester oils, an oily component such as an antiseptic or surfactant, and horse oil. Next, the aqueous phase part is gently stirred while being heated to about 80° C, at which point the oily phase part, which has been heated to almost the same temperature, is gradually added to the heated aqueous phase part to prepare ointment.

The melanogenesis-inhibiting endermic preparation of the present invention may be any one of the

above-mentioned forms, which contain a determined amount of a 6-hydroxychroman derivative of the above-mentioned formula.

Next, the present invention will be explained in more detail by way of the following examples, which, however, are not intended to restrict the scope of the present invention.

EXAMPLE 1 (Milky lotion):

| | | (% by weight) |
|---|---|---|
| (A) | Polyoxyethylene Glycol Monostearate (40 E.O.) | 2.00 |
| | Self-Emulsifying Glycerin Monostearate | 5.00 |
| | Stearic Acid | 5.00 |
| | Behenyl Alcohol | 1.00 |
| | Liquid Paraffin | 1.00 |
| | Glycerin Trioctanoate | 10.00 |
| | 2,2,5,7,8-Pentamethyl-6-hydroxychroman | 0.50 |
| | Antiseptic | ad lib. |
| | Perfume | trace |
| (B) | 1,3-Butylene Glycol | 5.00 |
| | Pure Water | balance |

The components of (A) are heated and dissolved to prepare an oily phase. Separately, the components of (B) are heated and dissolved to prepare an aqueous phase.

The aqueous phase is added to the oily phase, stirred, emulsified and then cooled to obtain a milky lotion.

EXAMPLE 2 (Lotion):

| | | (% by weight) |
|---|---|---|
| (A) | Polyoxyethylene Hardened Castor Oil (60 E.O.) | 1.00 |
| | Ethanol | 15.00 |
| | Citric Acid | 0.10 |
| | Sodium Citrate | 0.30 |
| | 1,3-Butylene Glycol | 4.00 |
| | 2,2,5,7,8-Pentamethyl-6-hydroxychroman | 0.50 |
| | Antiseptic | ad lib. |
| | Perfume | trace |
| | Pure Water | balance |

All of the above components are uniformly stirred, mixed and dissolved to prepare a lotion.

EXAMPLE 3 (Emulsion):

| | | (% by weight) |
|---|---|---|
| (A) | Polyethylene Behenyl Ether (20 E.O.) | 0.50 |
| | Polyoxyethylene Sorbitol Tetraoleate (60 E.O.) | 1.00 |
| | Oleophilic Glycerin Monostearate | 1.00 |
| | Stearic Acid | 0.50 |
| | Behenyl Alcohol | 0.50 |
| | Avocado Oil | 1.00 |
| | 2,2,5,7,8-Pentamethyl-6-hydroxychroman | 0.10 |
| | Antiseptic | ad lib. |
| | Perfume | trace |
| (B) | 1,3-Butylene Glycol | 5.00 |
| | Carboxyvinyl Polymer | 0.10 |
| | Pure Water | balance |

The components of (A) are heated and dissolved to prepare an oily phase. Separately, the components

of (B) are heated and dissolved to prepare an aqueous phase.

The aqueous phase is added to the oily phase, stirred, emulsified and cooled to prepare emulsion.

EXAMPLE 4 (Ointment):

| | | (% by weight) |
|---|---|---|
| (A) | Polyoxyethylene Sorbitan Monostearate (60E.O.) | 1.00 |
| | Polyoxyethylene Sorbitol Tetraoleate (60E.O.) | 1.50 |
| | Self-Emulsifying Glycerin Monostearate | 1.50 |
| | Bleached Bee's Wax | 2.00 |
| | Paraffin | 2.00 |
| | Stearic Acid | 3.00 |
| | Behenyl Alcohol | 3.00 |
| | Liquid Paraffin | 5.00 |
| | 2,2,5,7,8-Pentamethyl-6-hydroxychroman | 1.00 |
| | Antiseptic | ad lib. |
| | Perfume | trace |
| (B) | 1,3-Butylene Glycol | 5.00 |
| | Citric Acid | 0.30 |
| | Pure Water | balance |

The components of (A) are heated and dissolved to prepare an oily phase. Separately, the components of (B) are heated and dissolved to prepare an aqueous phase.

The aqueous phase is added to the oily phase, stirred, emulsified and cooled to prepare ointment.

EXAMPLE 5 (Cataplasm):

| | | (% by weight) |
|---|---|---|
| (A) | Polyacrylic Acid | 30.00 |
| | 2,2,5,7,8-Pentamethyl-6-hydroxychroman | 10.00 |
| | Sorbitan Monooleate | 1.00 |
| | Pure Water | 30.70 |
| (B) | Sodium Polyacrylate | 7.00 |
| | Aluminum Chloride | 0.30 |
| | Concentrated Glycerin | 20.00 |
| | Titanium Oxide | 1.00 |

The components of (A) are heated and dissolved. Separately, the components of (B) are heated and dissolved, and the resulting mixture of (B) is added to the mixture of (A). The whole is stirred and mixed to prepare a cataplasm.

EXPERIMENTAL EXAMPLE:

Evaluation of Melanogenesis-Inhibiting Effect of 6-Hydroxy-chroman Derivatives:

Test Compound:

2,2,5,7,8-Pentamethyl-6-hydroxychroman (referred to as PMC):

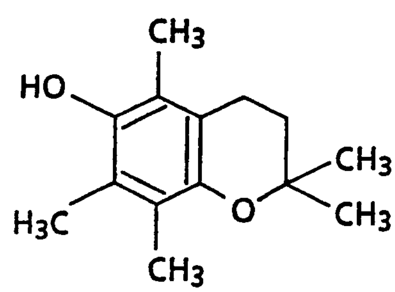

Test of Whitening B16 Cells:

PMC was added to Eagle's MEM medium, to which a fetal calf serum had been added, in a concentration of 100, 50, 20 or 10 μM. 1 × $10^5$ B16 cells were inoculated into the medium. Three days after the inoculation, the medium was exchanged for a fresh one. After five days, cell pellets were prepared, and the whiteness of the cells was observed with the naked eye.

Test Result and Conclusion:

The test result of whitening B16 cells by PMC is shown in Table 1 below, from which it is obvious that PMC is effective for whitening B16 cells in a concentration of from 50 to 100 μM.

Table 1 Test Result of Whitening B16 Cells by PMC

| | Concentration (μM) | Whiteness of Cells |
|---|---|---|
| PMC | 100 | 4 + |
| | 50 | 3 + |
| | 20 | 2 + |
| | 10 | 1 + |
| Control | | − |

Standards of evaluation of whiteness of cells are as follows:

5 + : White
4 + : White to Gray
3 + : Gray
2 + : Gray to Black
1 + : Black (but somewhat paler than control)
- : Black

The same tests were repeated, using the following compounds, whereupon the concentration of the test compound to give whiteness of cells of being 3 + (gray) was determined.

| Compound Tested | Concentration (μM) | Whiteness of Cells |
|---|---|---|
| (1) 2,2,5,7,8-Pentamethyl-6-hydroxychroman | 50 | 3 + |
| (2) Methyl 2,5,7,8-tetramethyl-6-hydroxychroman-2-carboxylate | 30 | 3 + |
| (3) 2,5,7,8-Tetramethyl-2-methoxzy-6-hydroxychroman | 50 | 3 + |
| (4) 2,2,8-Trimethyl-5,7-diisopropyl-6-hydroxychroman | 20 | 3 + |
| (5) 2,5,7,8-Tetramethyl-6-hydroxy-2-hydroxymethylchroman | 40 | 3 + |
| (6) Ethyl 2,5,7,8-tetramethyl-6-hydroxychroman-2-acetate | 30 | 3 + |

As is obvious from the above, the present invention provides a melanogenesis-inhibiting endermic preparation for external application which contains a 6-hydroxychroman derivative of the above-mentioned formula as an active ingredient, which also has an improved skin-whitening effect. The preparation of the present invention is safe to human use with no harmful side effects.

While the invention has been described in detail and with reference to specific embodiments thereof, it will be apparent to one skilled in the art that various changes and modifications can be made therein without departing from the spirit and scope thereof.

**Claims**

1. A melanogenesis-inhibiting endermic preparation for external application which contains, as an active ingredient, a 6-hydroxychroman derivative of a general formula:

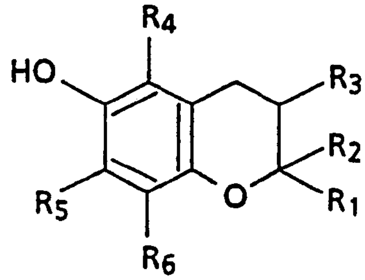

wherein $R_1$ represents a hydrogen atom, a lower alkyl group having from 1 to 6 carbon atoms, a lower alkoxy group having from 1 to 6 carbon atoms, a carboxylate residue of $-CO_2R'$ (wherein R' represents a lower alkyl group having from 1 to 6 carbon atoms), an alcohol residue of $-(CH_2)_nOH$ (wherein n represents an integer of from 1 to 6), an acetate residue of $-CH_2CO_2R'$ (wherein R' represents a lower alkyl group having from 1 to 6 carbon atoms), or a carboxylic acid amide residue of - CONHR' (wherein R' represents a lower alkyl group having from 1 to 6 carbon atoms); and $R_2$ to $R_6$ independently represent a hydrogen atom or a lower alkyl group having from 1 to 6 carbon atoms.

2. The melanogenesis-inhibiting endermic preparation for external application as claimed in claim 1, in which the 6-hydroxychroman derivative is 2,2,5,7,8-pentamethyl-6-hydroxychroman, 2,2,7,8-tetramethyl-6-hydroxychroman, 2,5,7,8-tetramethyl-2-methoxy-6-hydroxychroman, 2,2,8-trimethyl-5,7-diisopropyl-6-hydroxychroman, 2,5,7,8-tetramethyl-6-hydroxy-2-hydroxymethylchroman, 2,5,7,8-tetramethyl-6-hydroxychroman, methyl 2,5,7,8-tetramethyl-6-hydroxychroman-2-carboxylate, or ethyl 2,5,7,8-tetramehtyl-6-hydroxychroman-2-acetate.

3. The melanogenesis-inhibiting endermic preparation for external application as claimed in claim 1, in which the 6-hydroxychroman derivative is 2,2,5,7,8-pentamethyl-6-hydroxychroman.

European Patent Office

# EUROPEAN SEARCH REPORT

Application Number

EP 91103315.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | FR - A1 - 2 634 120 (R. BONIFACE) * Claim 2; page 2, line 4 - page 4, line 1 * -- | 1,2 | A 61 K 7/48<br>A 61 K 31/35 |
| X | EP - A1 - 0 238 302 (CHARLES OF THE RITZ GROUP) * Page 2, line 2 - page 3, line 18 * ---- | 1,2 | |
| | | | TECHNICAL FIELDS SEARCHED (Int. Cl.5) |
| | | | A 61 K 7/00<br>A 61 K 31/00 |

The present search report has been drawn up for all claims

| Place of search | Date of completion of the search | Examiner |
|---|---|---|
| VIENNA | 31-05-1991 | IRMLER |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document